# EUROPÄISCHE PATENTSCHRIFT

(11) **EP 2 109 430 B1**
(45) Veröffentlichungstag und Bekanntmachung des Hinweises auf die Patenterteilung: **04.03.2020**
(21) Anmeldenummer: 08701111.0
(22) Anmeldetag: 15.01.2008
(51) Int. Cl.: A61F 13/496, A61F 13/49, A61F 13/15

(54) **WEGWERFBARER HYGIENEARTIKEL IN PANTFORM MIT VERBESSERTER PASSFORM**
DISPOSABLE HYGIENE ITEM IN THE FORM OF A PAIR OF KNICKERS, WITH AN IMPROVED FIT
ARTICLE HYGIÉNIQUE JETTABLE EN FORME DE CULOTTE À AJUSTEMENT AMÉLIORÉ

(30) Priorität: 16.01.2007 DE 102007002290
(43) Veröffentlichungstag der Anmeldung: 21.10.2009
(73) Patentinhaber: Paul Hartmann AG, 89522 Heidenheim (DE)
(72) Erfinder: HORNUNG, Fridmann, Santiago (CL); OSTERTAG, Wolfgang, 89547 Gerstetten (DE); KAUTZSCH, Florian, North Caldwell, NJ 07006 (US); WENZEL, Benjamin, 89522 Heidenheim (DE)
(74) Vertreter: Oltmann, Eckhard
(86) Internationale Anmeldenummer: PCT/EP2008/000254
(87) Internationale Veröffentlichungsnummer: WO 2008/087006

(56) Entgegenhaltungen:
- EP-A- 0 626 161
- EP-A1- 1 970 036
- EP-A1- 2 011 464
- WO-A-96/34588
- JP-A- H08 280 738
- JP-A- 2003 284 737
- US-A- 5 622 581
- US-A- 5 634 917
- US-A1- 2006 161 128
- US-B1- 6 217 690

## Beschreibung

Die Erfindung betrifft einen wegwerfbaren absorbierenden Hygieneartikel für Erwachsene in Pantform, mit einem eine Hüftöffnung bildenden in Umfangsrichtung durchgehend geschlossenen Hüftrand und mit Beinöffnungen, wobei der in Umfangsrichtung durchgehend geschlossene Hüftrand und die Beinöffnungen gebildet sind, indem Längsseitenrandabschnitte eines Vorderteils und eines Rückenteils herstellerseitig miteinander verbunden sind, und mit einem Absorptionskern.

Derartige absorbierende Hygieneartikel in Höschenform sind bekannt. Sie umfassen zumeist eine Vielzahl von Elastifizierungsmitteln, häufig in Form von elastischen Fäden, die im vorgespannten Zustand mit im Wesentlichen unelastischen Chassismaterialien zumeist klebend verbunden werden. Dabei ist üblicherweise ein Hüftrandbereich vorzugsweise durchgehend in Umfangsrichtung elastifiziert. Auch im Vorderbereich und im Rückenbereich sind bei bekannten Windelhosen Elastifizierungsmittel vorgesehen. Desgleichen werden die Beinöffnungen umgebende bzw. die Beinöffnungen bildende Umfangsbereiche zumindest abschnittsweise elastisch ausgestaltet, damit dort ein weitgehend dichtendes Anliegen des Hygieneartikels an die Hautoberfläche des Benutzers gewährleistet ist, um das seitliche Austreten von Körperausscheidungen zu verhindern. Auch aufstehende Bündchenelemente, die neben den elastischen Beinöffnungen einen weiteren seitlichen Auslaufschutz bieten, werden bei bekannten Windelhosen bereits eingesetzt und sind (beispielsweise aus EP-1184017-A1, EP-1199058-A1, EP-1308148-A2) bekannt.

Es ist ferner bekannt, zwischen Hüftrand und Schrittbereich im Wesentlichen in einer Querrichtung erstreckte Elastifizierungsmittel vorzusehen, um die Passform des Hygieneartikels im Sinne einer körpernahen Anordnung zu verbessern. Des Weiteren ist bekannt geschwungene Elastifizierungsmittel vorzusehen, die sich von einer Beinöffnung zur anderen Beinöffnung erstrecken. Hierbei können auch Richtungswechsel vorgesehen sein (siehe beispielsweise US2006/0161128A1 WO96/34588A1, JP2003284737A, JPH08280738A, EP1970036A1, EP2011464A1).

Trotz des Einsatzes mehrerer elastischer Komponenten konnten die Passformprobleme von pantförmigen Einweghygieneartikeln für Erwachsene gleichwohl noch nicht zufriedenstellend gelöst werden. Gerade im Schrittbereich, also dort wo Passform und Zuverlässigkeit der Flüssigkeitsabsorption konfliktierende Zielgrößen darstellen, können die vorbekannten Hygieneartikel bislang keine zufriedenstellenden Lösungen anbieten.

Hiervon ausgehend liegt der vorliegenden Erfindung die Aufgabe zugrunde, den Tragekomfort absorbierender Hygieneartikel der eingangs genannten Art zu verbessern.

Diese Aufgabe wird durch einen Hygieneartikel für Erwachsene in Pantform mit den Merkmalen des Anspruch 1 gelöst.

Durch das Zusammenwirken der den zweiten Beinöffnungsabschnitt elastifizierenden faden- oder bandförmigen Elastifizierungsmittel und des elastischen Elements wird wirkungsvoll verhindert, dass die chassisbildende Hülle bei pantförmigen Hygieneartikeln für Erwachsene mit sehr schmalem Absorptionskern dort, wo sie besonders stark zur nicht gewünschten Faltenbildung neigt, nämlich im hinteren Schrittbereich seitlich außerhalb des Absorptionskerns, schräg nach hinten in Richtung Rückenteil gezwungen wird. Es hat sich überraschenderweise gezeigt, dass dies bei der überwiegenden Zahl der Benutzer der Passform des Hygieneartikels deutlich zugute kommt.

Dadurch, dass das elastische Element durch eine Weitererstreckung der zweiten faden- oder bandförmigen Elastifizierungsmittel gebildet ist, kann sich zudem im an den Benutzer angelegten Zustand zwischen den Schenkeln der U- oder V-förmigen Konfiguration eine nur einseitig geöffnete Tasche bilden, die zur Aufnahme von über den Seitenrand des Absorptionskerns übertretenden Flüssigkeiten und Fäkalien dienen kann.

Die Breite des Absorptionskerns beträgt auf Höhe der Quermittelachse insbesondere mindestens 10 cm, weiter insbesondere mindestens 12 cm und weiter insbesondere mindestens 13 cm, jedoch höchstens 18 cm, weiter insbesondere höchstens 17 cm, weiter insbesondere höchstens 16 cm, weiter insbesondere höchstens 15 cm und weiter insbesondere höchstens 14 cm, ist also vergleichsweise schmal ausgebildet.

Unter dem Absorptionskern wird im Rahmen der vorliegenden Erfindung der zur dauerhaften Aufnahme und Speicherung der ausgeschiedenen Körperflüssigkeiten bestimmte Teil des Hygieneartikels verstanden. Der Absorptionskern enthält üblicherweise superabsorbierende Materialien (SAP), insbesondere in Mischung mit Fasern, insbesondere Zellstofffasern, insbesondere in Form von Zellstofffluff. Falls der Hygieneartikel einen mehrteiligen oder mehrschichten Saugkörper umfasst, wird als Absorptionskern diejenige Schicht oder derjenige Teil des Saugkörpers angesehen, die/der den überwiegenden Teil der ausgeschiedenen Körperflüssigkeit bestimmungsgemäß dauerhaft speichert.

Eine weitere Ausbildung der Erfindung sieht vor, dass der hintere Schrittbereich einen an die Quermittelachse unmittelbar angrenzenden inneren Bereich und einen an den inneren Bereich anschließenden, sich in Längsrichtung bis zum Rückenteil erstreckenden äußeren Bereich aufweist, und wobei sowohl die zweiten faden- oder bandförmigen Elastifizierungsmittel, welche entlang einem zweiten Abschnitt der jeweiligen Beinöffnung angeordnet sind, als auch das elastische Element ausschließlich in dem äußeren Bereich angeordnet sind. In einem äußeren Bereich des hinteren Schrittbereiches weist die chassisbildende Hülle üblicherweise eine größere Breite auf als in einem inneren Bereich des Schrittbereiches. Das oben beschriebene Problem der Faltenbildung der chassisbildenden Hülle seitlich außerhalb des Absorptionskerns ist dort somit besonders ausgeprägt. In Weiterbildung dieses Erfindungsgedankens beträgt der Abstand in Querrichtung zwischen dem Rand des Absorptionskerns und einer jeweiligen Beinöffnung in dem äußeren Bereich zumindest 5 cm, insbesondere zumindest 7 cm und weiter insbesondere zumindest 9 cm.

In einer bevorzugten Ausführungsform der Erfindung beträgt die Länge der Längskomponente des elastischen Elementes 4,5 bis 19,0 cm, insbesondere 6,5 bis 17,0 cm und weiter insbesondere 7,5 bis 15,0 cm. Des Weiteren beträgt die Länge der Querkomponente des elastischen Elementes vorzugsweise 4,0 bis 15,0 cm, weiter insbesondere 5,0 bis 13,0 cm und weiter insbesondere 6,0 bis 12,0 cm.

In Weiterbildung des Erfindungsgedankens ist vorteilhaft, dass der innere Bereich des hinteren Schrittbereiches einer jeweiligen Beinöffnung dritte faden- oder bandförmige Elastifizierungsmittel aufweist, welche entlang einem dritten Abschnitt der jeweiligen Beinöffnung angeordnet sind. Diese dritten faden- oder bandförmigen Elastifizierungsmittel sind dabei ausschließlich in dem inneren Bereich des hinteren Schrittbereiches angeordnet.

Die die Beinöffnungsabschnitte elastifizierenden band- oder fadenförmigen Elastifizierungsmittel können insbesondere aus einer Schar von 2-7, insbesondere 2-6, weiter insbesondere 2-5 und ganz besonders 2-4 besonders dünner, in engem Abstand von 1-20 mm, insbesondere von 2-15 mm, weiter insbesondere von 3-10 mm und noch weiter insbesondere von 5-7 mm nebeneinander verlaufender Elastikfäden wie Lycra®- oder Spandex®-Fäden einer Stärke von 300-1500 dtex, insbesondere von 500-900 dtex gebildet sein, welche unter Vorspannung von 1,5-4,5, insbesondere von 2,0-4,0 an dem chassisbildenden Hüllmaterial fixiert sind.

Die Erstreckung der ersten, zweiten und dritten elastifizierten Abschnitte einer jeweiligen Beinöffnung in Längsrichtung des Hygieneartikels ist insbesondere derart gewählt, dass die Summe der Längen der ersten bis dritten Abschnitte einer jeweiligen Beinöffnung 20-60 cm, insbesondere 25-55 cm, weiter insbesondere 27-50 cm und weiter insbesondere 29-48 cm beträgt.

In Weiterbildung dieses Erfindungsgedankens hat es sich als dem Tragekomfort weiter zuträglich erwiesen, wenn das Verhältnis der Summe der Längen der ersten bis dritten Abschnitte einer jeweiligen Beinöffnung zur Umfangslänge einer jeweiligen Beinöffnung höchstens 0,7, insbesondere höchstens 0,6, weiter insbesondere höchstens 0,55 und weiter insbesondere höchstens 0,5 beträgt.

In Weiterbildung der Erfindung ist vorgesehen, dass die ersten, zweiten und dritten faden- oder bandförmigen Elastifizierungsmittel in Längsrichtung derart voneinander beabstandet sind, dass zwischen ersten und dritten und zwischen zweiten und dritten faden- oder bandförmigen Elastifizierungsmitteln jeweils ein nicht elastifizierter Beinöffnungsabschnitt vorgesehen ist. Vor dem Hintergrund, dass moderne Hygieneartikel zusätzlich über im Wesentlichen in Längsrichtung verlaufende aufstehende Bündchenelemente, so genannte "Cuff-Elemente" verfügen, die eine seitliche Barriere für das Austreten von Flüssigkeiten und auch festen Körperausscheidungen bilden, erscheint es als nicht mehr zwingend erforderlich, die Beinöffnungen in Umfangsrichtung durchgehend zu elastifizieren. Da Elastifizierungsmittel eine Raffung oder Rüschung von mit ihnen verbundenen Materialien bewirken, wenn sie im vorgespannten Zustand mit diesen Materialien verbunden werden, führen sie im relaxierten Zustand zu einer Materialanhäufung, die beispielsweise in sitzender Position auch als unangenehm empfunden werden kann. Jedenfalls reiben insbesondere bei Bewegung des Benutzers geraffte oder gerüschte Bereiche, also elastifizierte Bereiche, im besonderen Maße gegen die Hautoberfläche des Benutzers, was zu Hautirritationen führen kann. Durch die nicht elastifizierten Beinöffnungsabschnitte wird somit ein insgesamt besserer Tragkomfort erzielt.

Es hat sich als zweckmäßig und vorteilhaft erwiesen, wenn die ersten Elastifizierungsmittel von den dritten Elastifizierungsmitteln in Längsrichtung weiter beabstandet sind als die zweiten Elastifizierungsmittel von den dritten Elastifizierungsmitteln, da die Gefahr der unerwünschten Materialanhäufung im Zentrum des Schrittbereiches als besonders hoch angesehen wird.

In besonders vorteilhafter Weise verhält sich der Verlauf der dritten Elastifizierungsmittel weitestgehend spiegelbildlich zu dem Verlauf des elastischen Elementes, wobei die Spiegelachse eine in Querrichtung mittig verlaufende Linie zwischen den dritten Elastifizierungsmitteln und dem elastischen Element ist. Überraschenderweise ist dies der Passform des Hygieneartikels im besonderen Maße zuträglich. Offenbar wird hierdurch ein Gleichgewicht der elastischen Kräfte erzielt.

Es erweist sich ferner als vorteilhaft, wenn die chassisbildende Hülle im Rückenteil und/oder im Vorderteil und insbesondere auch in einem an das Rückenteil und/oder Vorderteil angrenzenden Bereich des Schrittbereiches in Querrichtung verlaufende faden- oder bandförmige Elastifizierungsmittel aufweist. Hierdurch kann die Passform des Hygieneartikels im Sinne einer körpernahen Anordnung weiter verbessert werden. Ein solches Elastifizierungsmittel kann im Bereich des Absorptionskörpers zu einer gewollten Zusammenziehung des Saugkörpers im Schrittbereich führen. Falls keine Raffung des Absorptionskerns gewünscht ist, können diese Elastifizierungsmittel insoweit sie unterhalb des Absorptionskerns verlaufen ihrer elastifizierenden Wirkung benommen, insbesondere durchtrennt werden. Die Elastifizierungsmittel werden dann relaxieren bzw. zurückschnellen, soweit sie in diesem Abschnitt keine Verbindung zur äußeren Hülle aufweisen. Die Durchtrennung kann beispielsweise durch einen einzigen Schnitt erfolgen. Es wäre aber auch denkbar und vorteilhaft, dass die Durchtrennung der Elastifizierungsmittel durch eine Vielzahl von Schnitten erfolgt, so dass die zuvor durchgehend erstreckten Elastifizierungsmittel in eine Vielzahl von kleinen Abschnitten, insbesondere mit einer Länge im Millimeterbereich, geteilt werden und so ihre elastifizierende Wirkung verlieren. Solchenfalls können die Elastifizierungsmittel auch klebend mit der äußeren Hülle verbunden sein, ohne dass dies die Relaxierung behindern würde. Ein einfaches oder mehrfaches Durchtrennen der Elastifizierungsmittel kann auch mittels Lasertechnik erfolgen. In einer alternativen Ausführungsform werden die zuvor erstreckten Elastifizierungsmittel durch Anwendung von Hitze und/oder Druck und/oder Ultraschall ihrer elastischen Wirkung benommen.

In einer besonders bevorzugten Ausführungsform ist vorgesehen, dass der hintere Schrittbereich in Längsrichtung eine größere Erstreckung aufweist als der vordere Schrittbereich. Wenn hier, zuvor oder im Folgenden von einem hinteren und/oder vorderen Schrittbereich die Rede ist, so liegt dem folgendes Verständnis zu Grunde: In Längsrichtung betrachtet umfasst der erfindungsgemäße Hygieneartikel wie zuvor beschrieben eine chassisbildende äußere Hülle mit einem Vorderteil, einem Rückenteil und einem zwischen Vorderteil und Rückenteil angeordneten die Beinöffnungen bildenden Schrittbereich. Vorderteil und Rückenteil sind diejenigen Chassisbereiche, deren Längsseitenrandabschnitte herstellerseitig zur Bildung der geschlossenen Pantform miteinander verbunden sind. Der Schrittbereich lässt sich durch eine in Querrichtung verlaufende Quermittelachse gedanklich in einen vorderen Schrittbereich und einen hinteren Schrittbereich teilen, wobei die Quermittelachse in Querrichtung durch das Minimum, das heißt die geringste Breite des Schrittbereiches verläuft. Bei Hygieneartikeln in Pantform, deren Schrittbereich rechteckförmig konfiguriert ist, wird die Quermittelachse so gelegt, dass diese den rechteckförmigen Bereich in Längsrichtung halbiert. Bei Hygieneartikeln in Pantform, die einer Bestimmung der Lage der Quermittelachse weder nach der ersten noch nach der zweiten Methode zulassen, erfolgt die Bestimmung der Lage der Quermittelachse nach der in EP0969784B1 mit Bezug zu Figur 4 dort im Abschnitt [0038] beschriebenen Methode. Hierbei wird der Schrittpunkt ("crotch point") bestimmt. Die Quermittelachse liegt dann auf Höhe dieses Schrittpunktes. Die Länge des hinteren Schrittbereiches ist somit der Teil, welcher sich in Längsrichtung betrachtet ausgehend von der Quermittelachse bis zum Beginn des Rückenteils erstreckt. Die Länge des vorderen Schrittbereichs ist analog, die Erstreckung des Schrittbereiches ausgehend von der Quermittelachse bis zum Beginn des Vorderteils.

In Weiterbildung dieses Erfindungsgedankens hat es sich hinsichtlich der Passform des Hygieneartikels als vorteilhaft erwiesen, wenn der hintere Schrittbereich eine Länge von 20-50 cm, insbesondere eine Länge von 22-45 cm und weiter insbesondere eine Länge von 26-39 cm aufweist. In besonders vorteilhafter Weise weist der innere und/oder der äußere Bereich des hinteren Schrittbereichs eine Länge von 8-30 cm, insbesondere von 10-25 cm und weiter insbesondere von 12-22 cm auf.

Der Passform des Hygieneartikels ist es weiter zuträglich, wenn dass Verhältnis der Länge des inneren Bereiches des hinteren Schrittbereiches zur Länge des äußeren Bereiches des hinteren Schrittbereiches zwischen 0,4 und 0,6, insbesondere zwischen 0,45 und 0,55 und weiter insbesondere zwischen 0,47 und 0,52 liegt.

In einer weiteren vorteilhaften Ausführungsform der Erfindung ist vorgesehen, dass die ersten und/oder zweiten und/oder dritten faden- oder bandförmigen Elastifizierungsmittel ausgehend von einer ersten Beinöffnung den Schrittbereich quer unterhalb des Absorptionskerns traversieren, um auf der gegenüberliegenden Seite einen zur Längsmittelachse spiegelbildlichen Verlauf entlang der weiteren zweiten Beinöffnung zu nehmen. Solchenfalls kann der Saugkörper im Schrittbereich eine Raffung erfahren. Es kann hingegen in vorteilhafter Weise ebenfalls vorgesehen sein, dass die unterhalb des Absorptionskerns verlaufenden Elastifizierungsmittel insbesondere durch Anwendung von Hitze und/oder Druck und/oder Lasereinwirkung und/oder Ultraschall und/oder durch Schneiden zumindest abschnittsweise ihrer elastifizierenden Wirkung benommen worden sind, oder die unterhalb des Absorptionskerns verlaufenden Elastifizierungsmittel spannungsfrei verlaufen. Solchenfalls kann eine unerwünschte Raffung des Absorptionskerns vermieden werden.

Vorteilhafterweise können beidseits des Absorptionskerns und in dessen Längsrichtung erstreckt Bündchenelemente mit einem elastischen Bündchenabschnitt vorgesehen sein. Derartige aufstehende Bündchenelemente, die neben den elastischen Beinöffnungen einen weiteren seitlichen Auslaufschutz bieten, werden bei bekannten Hygieneartikeln in Pantform bereits eingesetzt und sind beispielsweise aus EP1184017, EP1199058 oder EP1308148 bekannt.

In einer bevorzugten Ausführungsform ist der Absorptionskern mit seiner körperfernen Seite an der körpernahen Schicht der chassisbildenden äußeren Hülle angeordnet und vorzugsweise daran durch Klebemittel, wie Heißschmelzklebemittel oder andere Fügeverfahren, wie thermisches Schweißen, zumindest abschnittsweise fixiert.

Der Absorptionskern umfasst Körperflüssigkeiten absorbierende Materialien wie natürliche oder synthetische Fasern, insbesondere Zellulosefasern, vorzugsweise in Form von Zellstofffluff. Vorzugsweise umfasst der Absorptionskern außerdem superabsorbierende Materialien (SAP), insbesondere auf Basis oberflächenvernetzter, teilneutralisierter Polyacrylate.

Der Absorptionskern wird vorzugsweise von einem zumindest abschnittsweise flüssigkeitsdurchlässigen Topsheet überfangen und weiter vorzugsweise von einem zumindest abschnittsweise im Gebrauch flüssigkeitsundurchlässigem Backsheet unterfangen. Das Topsheet umfasst insbesondere Vliesstoffmaterialien oder mit Öffnungen versehenen Folienmaterialien. Das Backsheet umfasst insbesondere eine Folie, insbesondere einer Dicke von höchstens 15 µm. Das Backsheet kann vorteilhafter Weise aber auch im Gebrauch flüssigkeitsdichte gleichwohl aber wasserdampfdurchlässige Vliesstoffmaterialien wie Meltblown-Schichten (M) und Spunbond-Schichten (S), insbesondere Laminate aus Meltblown- und Spunbond-Schichten, wie SM- oder SMS- oder SMMS-Laminate enthalten oder daraus bestehen. Insbesondere umfasst das Backsheet eine im Gebrauch flüssigkeitsdichte aber gleichwohl atmungsaktive, also wasserdampfdurchlässige, insbesondere mikroporöse Folie. Die Wasserdampfdurchlässigkeit des Backsheets beträgt insbesondere wenigstens 300g/m²/24h, weiter insbesondere wenigstens 1000g/m²/24h, weiter insbesondere wenigstens 2000g/m²/24h, weiter insbesondere wenigstens 3000g/m²/24h, weiter insbesondere wenigstens 4000g/m²/24h, weiter insbesondere höchstens 6000g/m²/24h gemessen nach DIN 53 122-1 (Ausgabe: 2001-08).

Die optional vorgesehenen Bündchenelemente erstrecken sich beidseits des Absorptionskerns und sind insbesondere mit ihren proximalen Rändern an einem jeweiligen Längsrand der körpernahen Seite des Absorptionskerns, insbesondere dem Topsheet festgelegt. In einer alternativen Weiterbildung der Erfindung sind die proximalen Ränder der Bündchenelemente beidseits außerhalb des Absorptionskerns, insbesondere auf der körpernahen Seite der chassisbildenden äußeren Hülle festgelegt. Die distalen, zum Benutzer hin zumindest abschnittsweise aufstehenden Ränder weisen vorzugsweise Elastifizierungsmittel, insbesondere faden- oder bandförmige Elastifizierungsmittel der vorgenannten Art auf. In einer alternativen Ausführungsform umfassen oder bestehen die Bündchenelemente aus massiv elastischem Material, wie einer elastischen Folie oder einem elastischen Vliesstoff.

Vorzugsweise ist der distale Rand der Bündchenelemente nach innen in Richtung auf die Längsachse des Hygieneartikels geneigt. Dies wird insbesondere dadurch sichergestellt, dass der distale Rand der Bündchenelemente in einem oder beiden Endabschnitten nach innen auf der körperzugewandten Seite des Absorptionskerns, insbesondere auf das Topsheet festgelegt ist. Durch die Festlegung der Endabschnitte der Bündchenelemente über einen genügend großen Abschnitt auf dem Absorptionskern kann in einer bevorzugten Ausführungsform diesem Abschnitt gleichzeitig die elastische Wirkung genommen werden, womit dieser festgelegte Endabschnitt einen nicht elastifizierten Bündchenabschnitt bildet.

Nach einem weiteren an sich völlig unabhängigen Erfindungsgedanken weist der Hygieneartikel in Pantform im vorderen Schrittbereich an einer jeweiligen Beinöffnung einen hüftöffnungsnahen Bereich, einen schrittzentrumsnahen Bereich und einen dazwischen angeordneten mittleren Bereich auf, wobei der hüftöffnungsnahe Bereich, der schrittzentrumsnahe Bereich und der mittlere Bereich Elastifizierungsmittel aufweisen und wobei die Kräfteresultierende der Elastifizierungsmittel jeweils eine Komponente in Querrichtung aufweist und wobei diese Komponente in Querrichtung in dem hüftöffnungsnahen Bereich größer ist als in dem mittleren und/oder dem schrittzentrumsnahen Bereich, und die Komponente in Querrichtung in dem mittleren Bereich größer ist als in dem schrittzentrumsnahen Bereich. Insbesondere kann sich an den schrittzentrumsnahen Bereich bis zur Quermittelachse ein weiterer Bereich erstrecken, in dem die Beinöffnungen keinerlei Elastifizierungsmittel aufweisen. Hierdurch wird die Passform des Hygieneartikels in besonderer Weise begünstigt. Insbesondere sollten die Elastifizierungsmittel in dem hüftöffnungsnahen Bereich der Beinöffnungen ausschließlich durch im Wesentlichen in Querrichtung angeordnete faden- oder bandförmige Elastifizierungsmittel gebildet sein. In Weiterbildung dieses Erfindungsgedankens erweist es sich als vorteilhaft, wenn die Elastifizierungsmittel in dem mittleren Bereichen sowohl durch einen hüftöffnungsnahen Abschnitt der ersten faden- oder bandförmigen Elastifizierungsmittel als auch durch im Wesentlichen in Querrichtung angeordnete faden- oder bandförmige Elastifizierungsmittel gebildet sind. Hingegen sind die Elastifizierungsmittel in dem schrittzentrumsnahen Bereich insbesondere ausschließlich durch einen schrittzentrumsnahen Abschnitt der ersten faden- oder bandförmigen Elastifizierungsmittel gebildet.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform des erfindungsgemäßen Hygieneartikels. In der Zeichnung zeigt:
- Figur 1: eine Draufsicht auf eine Ausführungsform des erfindungsgemäßen Hygieneartikels im flachgelegten, ausgestreckten Zustand vor der herstellerseitigen Verbindung von Längsseitenrandbereichen
- Figur 2: eine Figur 1 entsprechende Darstellung mit Wiedergabe von den Absorptionskern einhüllendem Topsheet und Backsheet
- Figur 3: eine Schnittansicht der Figur 2 entlang A - A
- Figur 4: eine Figur 1 entsprechende Darstellung mit Kennzeichnung der Längen von Elastifizierungsm itteln
- Figur 5: eine weitere Figur 1 entsprechende Darstellung mit Kennzeichnung von Beinöffnungsbereichen
- Figur 6: eine perspektivische Ansicht eines erfindungsgemäßen Hygieneartikels nach herstellerseitiger Verbindung von Längsseitenrandbereichen

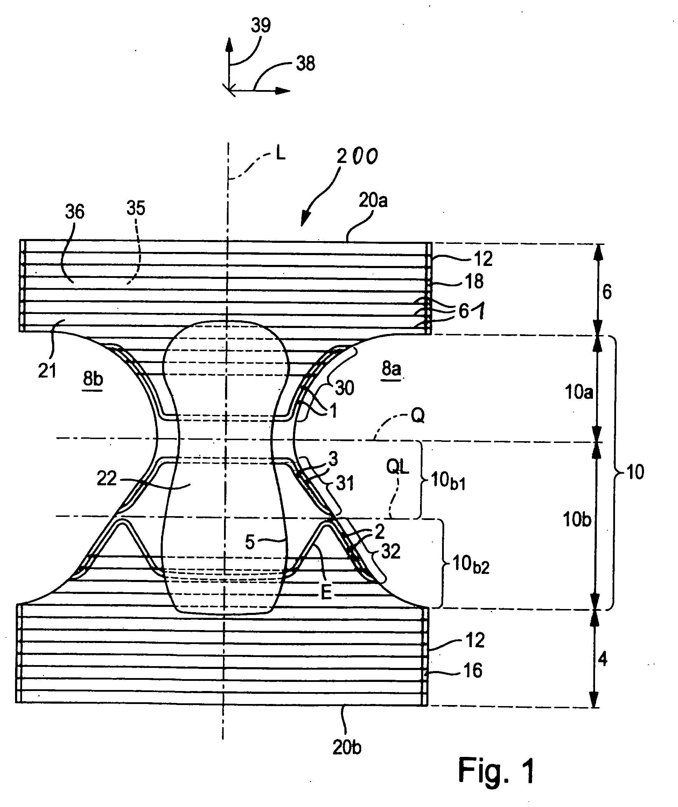

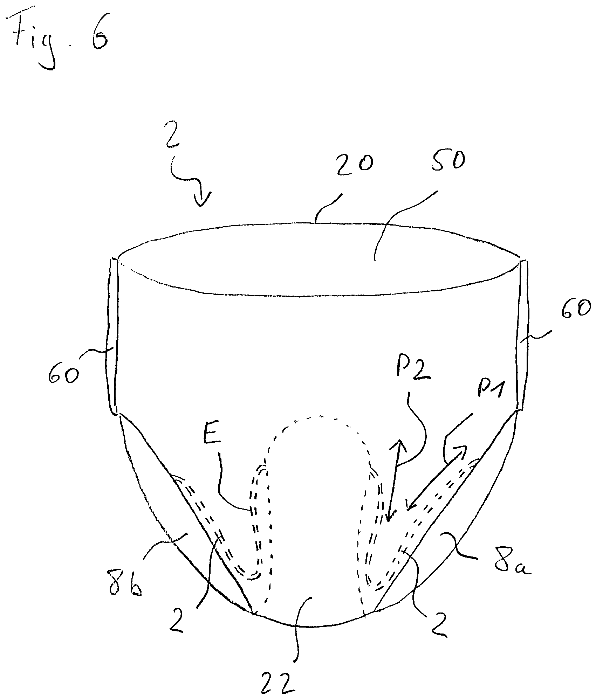

Figuren 1-5 zeigen einen insgesamt mit dem Bezugszeichen 200 bezeichneten Hygieneartikel für Erwachsene in Form einer Windelhose ("pant"), jedoch im flachgelegten, ausgestreckten Zustand vor der herstellerseitigen Verbindung der Längsseitenrandabschnitte 16, 18. Die Figuren 1-5 zeigen jeweils denselben Hygieneartikel, wobei der Übersicht halber jeweils unterschiedliche Details dargestellt sind. Es wird darauf hingewiesen, dass alle vorstehend und nachfolgend angegebenen und beanspruchten Abmessungen des Hygieneartikels sich auf diesen flachgelegten, ausgestreckten Zustand beziehen. Figur 6 zeigt den Hygieneartikel nach Verbindung der Längsseitenrandabschnitte 16, 18.

Der Hygieneartikel 200 umfasst eine äußere chassisbildende Hülle 21 mit einem Rückenteil 4, einem Vorderteil 6 und einem dazwischen liegenden, die späteren Beinausschnitte 8 a, b begrenzenden Schrittbereich 10. Im flachgelegten Zustand werden Rücken- und Vorderteil von Längsseitenrändern 12 begrenzt. Diese werden zur Bildung der Pant-Form im Bereich der Längsseitenrandabschnitte 16 bzw. 18 miteinander verbunden, etwa durch Heißsiegeln oder sonstige übliche Fügeverfahren. Auf diese Weise werden zwei Seitennahtbereiche 60 (Figur 6) des Hygieneartikels 200 gebildet, die nicht zerstörungsfrei gelöst werden können. Der fertig konfigurierte Hygieneartikel kann aber durchaus, insbesondere entlang dieser Seitennahtbereiche, eine Sollbruchlinie aufweisen, um den Hygieneartikel ausgehend vom angelegten Zustand öffnen zu können. Auch können zusätzlich an sich beliebige Verschlusselemente, etwa klebende oder mechanisch haftende Verschlusslaschen, vorgesehen sein, um den Hygieneartikel nach dem Öffnen wieder wie eine Windel der offenen Form schließen zu können. Herstellerseitig ist der Hygieneartikel aber als Höschen ("pant") gefertigt und weist daher einen aus dem vorderen Hüftrand 20a und dem hinteren Hüftrand 20b gebildeten, in Umfangsrichtung ausgeschlossenen Hüftrand 20 auf, der eine allseits begrenzte Hüftöffnung 50 bildet.

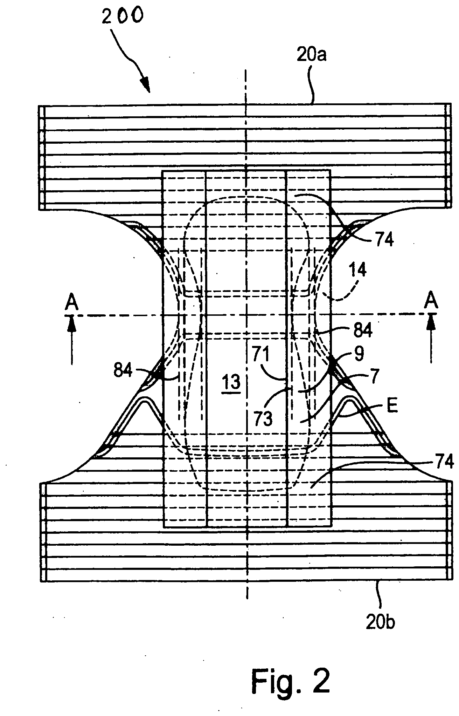

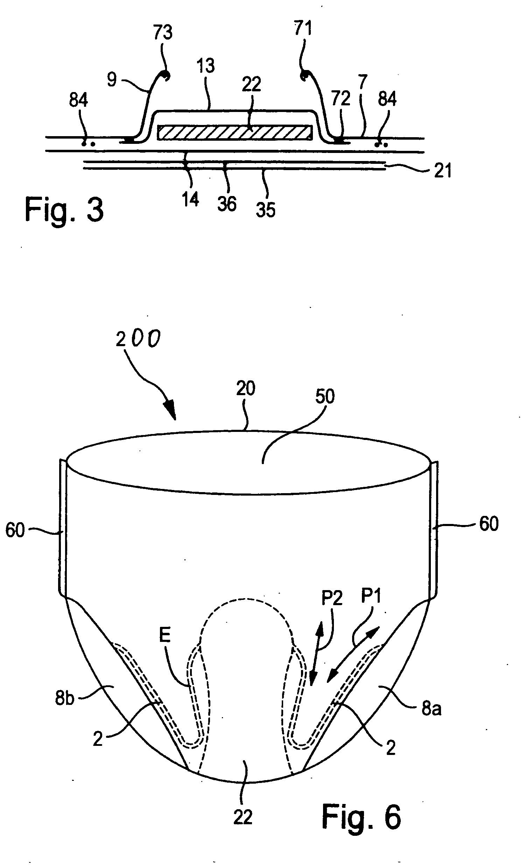

Der Hygieneartikel 200 umfasst des Weiteren einen Absorptionskern 22, der auf der dem Körper zugewandten Seite der äußeren Hülle 21 fixiert ist. Der Absorptionskern 22 weist, wie in Figuren 2 und 3, nicht aber in Figuren 1, 4 und 5 dargestellt, seinerseits ein flüssigkeitsundurchlässiges Backsheet 14 und ein flüssigkeitsdurchlässiges Topsheet 13 auf.

Der Absorptionskern 22 ist als funktionale und vorgefertigte Einheit mit seiner körperfernen Seite, also mit der körperabgewandten Seite des Backsheets 14 auf der körpernahen Seite der äußeren Hülle 21 verbunden.

Die das Windelchassis bildende äußere Hülle 21 erstreckt sich kontinuierlich vom vorderen Hüftrand 20a zum hinteren Hüftrand 20b und umfasst vorliegend ein zweilagiges Vliesstofflaminat bestehend aus einer körpernahen unelastischen Vliesstoffschicht 36 und einer körperfernen unelastischen Vliesstoffschicht 35 mit einem Flächengewicht von je 22 g/m², die zumindest abschnittsweise verbunden sind. Unter einem unelastischen Material, wie Vliesstoff oder Folie, wird im Rahmen der vorliegenden Erfindung ein Material verstanden, das nach einer einmaligen Dehnung eines 25 mm breiten Materialstreifens um 30 % seiner Ausgangslänge bei einer Dehngeschwindigkeit von 500 mm/min entweder reißt oder bei anschließender sofortiger Entlastung eine permanente Dehnung von mindestens 7,5 % aufweist. Dies bedeutet, dass beispielsweise ein 100 mm langer Materialstreifen, der auf eine Länge von 130 mm gedehnt wurde nach Entlastung eine Länge von mindestens 107,5 cm aufweist.

Von einem jeweiligen Hüftrand 20a, 20b bis in den Schrittbereich 10 hinein erstrecken sich in Querrichtung 38 elastische Fäden 6, die zwischen den beiden Vliesstoffschichten 35, 36 vorgespannt durch Schmelzhaftklebstoff fixiert sind.

Das Rückenteil 4 des Hygieneartikels weist in Längsrichtung 39 eine Erstreckung von 17 cm auf. Die Länge des Vorderteils 6 beträgt ebenfalls 17 cm. Der zwischen Vorder- und Rückenteil liegende Schrittbereich 10 wird durch die Quermittelachse Q in einen hinteren Schrittbereich 10b und einen vorderen Schrittbereich 10a geteilt. Die Quermittelachse Q verläuft in Querrichtung 38, senkrecht zur Längsmittelachse L auf Höhe des Minimums der Breite des Schrittbereiches 10 der chassisbildenden Hülle 21. Die Längsmittelachse L ist eine gedachte Linie parallel zur Längsrichtung 39, welche die Quererstreckung der chassisbildenden Hülle 21, also deren dortige Breite, auf Höhe der Quermittellängsachse halbiert. Der vordere Schrittbereich 10a weist in Längsrichtung 39 eine Erstreckung von 17,5 cm auf. Der hintere Schrittbereich 10b weist in Längsrichtung 39 eine Erstreckung von 29 cm auf.

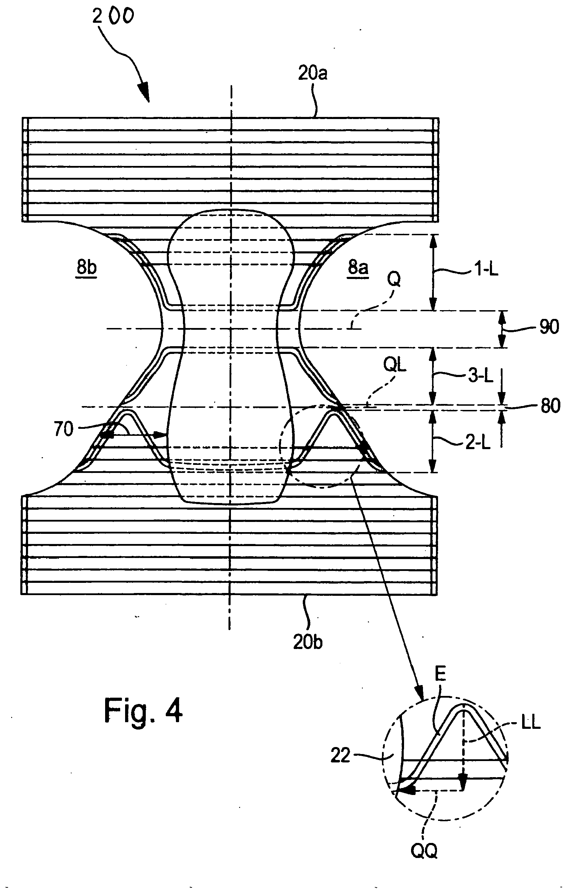

Jede Beinöffnung 8a, 8b weist entlang einem ersten Abschnitt 30 erste fadenförmige Elastifizierungsmittel 1 auf, welche in vorgespanntem Zustand zwischen den die chassisbildende Hülle 21 bildenden Vliesstofflagen 35, 36 fixiert sind. Diese ersten Elastifizierungsmittel 1 sind ausschließlich in dem vorderen Schrittbereich 10a angeordnet. Der hintere Schrittbereich 10b einer jeweiligen Beinöffnung 8 a, b weist in einem äußeren Bereich 10b2 entlang einem zweiten Abschnitt 32 zweite, von den ersten verschiedene fadenförmige Elastifizierungsmittel 2 auf, welche ebenfalls in vorgespanntem Zustand an der chassisbildenden Hülle fixiert sind. Diese zweiten Elastifizierungsmittel 2 verlaufen unter Ausbildung einer v-förmigen Konfiguration ausgehend von einem beinöffnungsnahen Bereich schräg nach hinten und bilden damit in dem äußeren Bereich 10b2 zwischen dem Längsrand 5 des Absorptionskerns und der Beinöffnung ein elastisches Element E, deren Erstreckung ausgehend von dem beinöffnungsnahen Bereich sowohl eine Querkomponente QQ, also eine Komponente in Querrichtung 38, in Richtung auf die Mittellängsachse L als auch eine Längskomponente LL, also eine Komponente in Längsrichtung 39, in Richtung auf das Rückenteil 4 aufweist (Figur 4). Die Länge der Querkomponente QQ beträgt 6,7 cm, die Lange der Längskomponente LL beträgt 9,0 cm. Das elastische Element E ist somit durch eine Weitererstreckung der zweiten Elastifizierungsmittel 2 gebildet. Die Wirkung dieser Anordnung von Elastiks ist in Figur 6 schematisch durch die Andeutung von Kräften P1 und P2 dargestellt, welche das Windelchassis in dem Bereich außerhalb des Absorptionskerns im kritischen, zur Faltenbildung neigenden Bereich glatt ziehen und damit eng an den Körper des Benutzers anlegen. Der kritische zur Faltenbildung neigende Bereich ist bei Hygieneartikeln in Pantform dort problembehaftet, wo der Abstand 70 (Figur 4) im hinteren Schrittbereich 10b, insbesondere im äußeren Bereich 10b2 des hinteren Schrittbereiches 10b zwischen Absorptionskern 22 und Beinöffnung 8 a, b in Querrichtung 38 größer ist als 5 cm, insbesondere größer als 7 cm und weiter insbesondere größer als 9 cm ist und damit relativ große Flächenabschnitte der chassisbildenden Hülle 21 der Gefahr der Faltenbildung ausgesetzt sind.

Die Breite des Absorptionskerns, also dessen Erstreckung in Querrichtung, auf Höhe der Quermittelachse Q beträgt lediglich 14 cm. Die Breite der chassisbildenden Hülle 21 beträgt dort 22 cm.

Der Hygieneartikel 200 weist im hinteren Schrittbereich 10b außerdem dritte, von den ersten und zweiten verschiedene fadenförmige Elastifizierungsmittel 3 auf, die entlang einem dritten Abschnitt 31 einer jeweiligen Beinöffnung 8a, 8b verlaufen. Diese dritten Elastifizierungsmittel 3 sind ausschließlich in einem inneren Bereich 10b1 des hinteren Schrittbereichs 10b angeordnet, während die zweiten Elastifizierungsmittel 2 ausschließlich in einem äußeren Bereich 10b2 des hinteren Schrittbereiches 10b angeordnet sind.

Erkennbar ist, dass eine Querachse QL die Grenze zwischen innerem 10b1 und äußerem Bereich 10b2 des hinteren Schrittbereiches 10b bildet. Die Querachse QL verläuft mittig zwischen den voneinander in Längsrichtung 39 um den Abstand 80 (Figur 4) voneinander beabstandeten zweiten und dritten Elastifizierungsmittel 2, 3 und bildet bezüglich des Verlaufes der dritten Elastifizierungsmittel 3 und dem elastischen Element E eine Spiegelachse. Der Abstand 80 beträgt hierbei 1,5 cm.

Der innere Bereich 10b1 des hinteren Schrittbereichs 10b weist in Längsrichtung 39 eine Erstreckung von 14,3 cm auf. Der äußere Bereich 10b2 des hinteren Schrittbereiches 10b weist in Längsrichtung 39 eine Erstreckung von 14,7 cm auf.

Erkennbar ist, dass die ersten, zweiten und dritten faden- oder bandförmigen Elastifizierungsmittel 1, 2, 3 ausgehend von der ersten Beinöffnung 8a den Schrittbereich 10 quer unterhalb des Absorptionskerns 22 traversieren, um auf der gegenüberliegenden Seite einen zur Längsmittelachse L spiegelbildlichen Verlauf entlang der weiteren zweiten Beinöffnung 8b zu nehmen. Unterhalb des Absorptionskerns 22 sind die Elastifizierungsmittel 1, 2, 3 insbesondere durch Anwendung von Hitze und/oder Druck und/oder Lasereinwirkung und/oder Ultraschall und/oder durch Schneiden zumindest abschnittsweise ihrer elastifizierenden Wirkung benommen. Denkbar wäre auch, dass die Elastifizierungsmittel 1, 2, 3 dort spannungsfrei verlaufen.

Die ersten Elastifizierungsmittel 1 sind von den dritten Elastifizierungsmitteln 3 in Längsrichtung 39 um den Abstand 90 von 6,0 cm beabstandet (Figur 4).

Die maximale Erstreckung der ersten, zweiten und dritten Elastifizierungsmittel in Längsrichtung 39 ist in Figur 4 dargestellt und dort mit 1-L, 2-L und 3-L bezeichnet. Ein bedeutender Anteil der Beinöffnungen 8a, 8b weist hierbei kein Elastifizierungsmittel mit Komponente in Längsrichtung 39 auf. Hierdurch wird bei immer noch ausreichender Elastifizierung der Beinöffnung sichergestellt, dass in Gebrauch keine übermäßige Faltenbildung mit der Folge von Hautirritationen erzeugt wird. Die Summe der Längen (1-L + 2-L + 3-L) beträgt lediglich 31,5 cm. Die Umfangslänge einer jeweiligen Beinöffnung, beträgt 68,9 cm. Die Umfangslänge einer jeweiligen Beinöffnung ist somit um 48,2% größer als die Erstreckung des Schrittbereiches 10 in der Längsrichtung 39, welche lediglich 46,5 cm beträgt. Das Verhältnis der Summe der Längen 1-L + 2-L + 3-L zur Länge des Umfangs einer jeweiligen Beinöffnung beträgt somit lediglich 0,46.

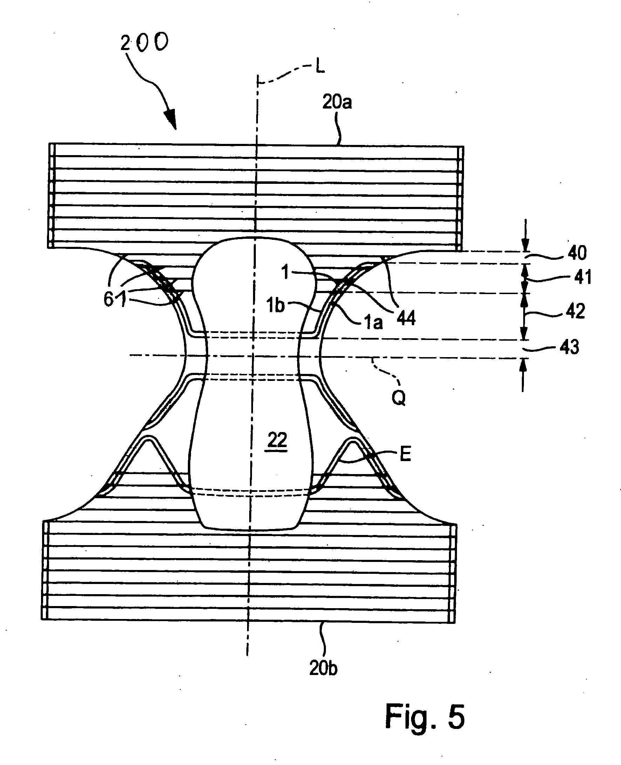

Figur 5 veranschaulicht, dass eine jeweilige Beinöffnung 8 a, b im vorderen Schrittbereich 10a drei unterschiedlich elastifizierte Bereiche aufweist, nämlich einen hüftöffnungsnahen Bereich 40, einen schrittzentrumsnahen Bereich 42 und einen dazwischen angeordneten mittleren Bereich 41, wobei der hüftöffnungsnahe Bereich 40, der schrittzentrumsnahe Bereich 42 und der mittlere Bereich 41 fadenförmige Elastifizierungsmittel 44 aufweisen. Fadenstärke, Vorspannung, Richtung und die Dichte der Anordnung der Elastifizierungsmittel 44 sind so gewählt, dass die Kräfteresultierende der Elastifizierungsmittel 44 jeweils eine Komponente in Querrichtung 38 aufweist und wobei diese Komponente in Querrichtung in dem hüftöffnungsnahen Bereich 40 größer ist als in dem mittleren Bereich 41, und die Komponente in Querrichtung in dem mittleren Bereich 41 größer ist als in dem schrittzentrumsnahen Bereich 42. Hierdurch ist die Passform des Hygieneartikels um das Bein herum in besonders vorteilhafter Weise sichergestellt. Im dargestellten Fall sind die Elastifizierungsmittel 44 in dem hüftöffnungsnahen Bereich 40 der Beinöffnungen 8a, b ausschließlich durch im Wesentlichen in Querrichtung angeordnete faden- oder bandförmige Elastifizierungsmittel 61 gebildet. Die Elastifizierungsmittel 44 in dem mittleren Bereichen 41 sind sowohl durch einen hüftöffnungsnahen Abschnitt 1a der ersten faden- oder bandförmigen Elastifizierungsmittel 1 als auch durch die im Wesentlichen in Querrichtung angeordnete faden- oder bandförmige Elastifizierungsmittel 61 gebildet sind. Schließlich sind die Elastifizierungsmittel 44 in dem schrittzentrumsnahen Bereich 42 ausschließlich durch einen schrittzentrumsnahen Abschnitt 1b der ersten faden- oder bandförmigen Elastifizierungsmittel 1 gebildet. An den schrittzentrumsnahen Bereich 42 schließt sich bis zur Quermittelachse Q ein weiterer Bereich 43 an, in welchem die Beinöffnung keinerlei Elastifizierungsmittel aufweist.

Als Schrittzentrum wird derjenige Abschnitt des Schrittbereiches 10 verstanden, welcher die Quermittelachse Q umfasst.

Figuren 2 und 3 zeigen schließlich in detailierterer Darstellung, dass der Absorptionskern 22 von einem flüssigkeitsundurchlässiges Backsheet 14 unterfangen und von einem flüssigkeitsdurchlässiges Topsheet 13 überfangen ist. Die Bündchenelemente 7 sind aus einem hydrophoben Vliesmaterial gebildet und sind mit ihrem proximalen Rand 72 beidseits des Absorptionskerns 22 parallel zur Mittellängsachse auf dem Topsheet 13 festgelegt. Der jeweilige distale Rand 71 der Bündchenelemente 7 ist in einem zumindest das Schrittzentrum umfassenden Abschnitt, dem elastischen Bündchenabschnitt 9, mit einem Elastifizierungsmittel, nämlich einem vorgespanntem elastischen Faden 73 versehen. Vermittels der Rückstellkraft des Fadens kann der elastische Bündchenabschnitt 9 wie Figur 3 zeigt nach oben gegen die Haut des Benutzers aufstehen. In Richtung des vorderen und hinteren Hüftrandes 20a, 20b verbleibt das jeweilige Bündchenelement unelastifiziert und bildet dort somit nicht elastifizierte Abschnitte. Der distale Rand 71 ist an einem hinteren und vorderen Endabschnitt 74 des Bündchenelementes 7 nach innen umgelegt und auf dem Topsheet 13 durch nicht dargestellte Ultraschallschweißpunkte festgelegt. Der Verbund umfasst außerdem parallel zur Mittellängsachse L orientierte Elastifizierungsmittel in Form von elastischen Fäden 84 die, wie Figur 3 zeigt, zwischen Topsheet und Backsheet fixiert sind. Vorzugsweise sind die Elastifizierungsmittel 84 in Querrichtung 38 betrachtet außerhalb des proximalen Randes 72 der Bündchenelemente 7 angeordnet, das heißt die elastischen Fäden 84 sind näher an den Beinöffnungen 8 a, b angeordnet als es die proximalen Ränder 72 der Bündchenelemente 7 sind.

## Patentansprüche

1. Wegwerfbarer absorbierender Hygieneartikel (200) für Erwachsene in Pantform mit einem Absorptionskern (22) und mit einer chassisbildenden äußeren Hülle (21) mit einem eine Hüftöffnung bildenden in Umfangsrichtung durchgehend geschlossenen Hüftrand (20), und mit Längsseitenrandabschnitte (16, 18) aufweisendem Vorderteil (6) und Rückenteil (4), und mit einem zwischen Vorderteil (6) und Rückenteil (4) angeordneten die Beinöffnungen (8 a, b) bildendem Schrittbereich (10), wobei der in Umfangsrichtung durchgehend geschlossene Hüftrand (20) und die Beinöffnungen (8 a, b) gebildet sind, indem Längsseitenrandabschnitte (16, 18) eines Vorderteils (6) und eines Rückenteils (4) der chassisbildenden äußeren Hülle (21) herstellerseitig miteinander verbunden sind, und mit einer in einer Längsrichtung erstreckten Mittellängsachse (L) und mit einer durch die Mitte des Schrittbereiches (10) in Querrichtung verlaufenden Quermittelachse (Q), welche den Schrittbereich in einen vorderen Schrittbereich (10a) und einen hinteren Schrittbereich (10b) teilt, und wobei eine jeweilige Beinöffnung (8 a, b) in dem vorderen Schrittbereich (10a) erste faden- oder bandförmige Elastifizierungsmittel (1) aufweist, welche entlang einem ersten Abschnitt (30) der jeweiligen Beinöffnung angeordnet sind, und wobei der hintere Schrittbereich (10b) zweite faden- oder bandförmige Elastifizierungsmittel (2) aufweist, welche entlang einem zweiten Abschnitt (32) der jeweiligen Beinöffnung (8 a, b) angeordnet sind, und wobei der hintere Schrittbereich (10b) zwischen dem jeweiligen Längsrand (5) des Absorptionskerns (22) und der jeweiligen Beinöffnung (8 a, b) ein elastisches Element (E) aufweist, welches ausgehend von einem beinöffnungsnahen Bereich in einer Richtung angeordnet ist, die sowohl eine Querkomponente (QQ) in Richtung auf die Mittellängsachse als auch eine Längskomponente (LL) in Richtung auf das Rückenteil (4) aufweist, **dadurch gekennzeichnet, dass** das elastische Element (E) durch eine Weitererstreckung der zweiten faden- oder bandförmigen Elastifizierungsmittel (2) gebildet ist, indem diese zunächst entlang dem zweiten Abschnitt (32) der jeweiligen Beinöffnung in Richtung der Quermittelachse (Q) verlaufen und anschließend unter Ausbildung einer u- oder v-förmigen Konfiguration ausgehend von dem beinöffnungsnahen Bereich in einer Richtung verlaufen, die sowohl eine Querkomponente QQ in Richtung auf die Mittellängsachse (L) als auch eine Längskomponente LL in Richtung auf das Rückenteil (4) aufweist, wobei die Länge der Längskomponente (LL) des elastischen Elementes (E) 2,5-21,0 cm und die Länge der Querkomponente (QQ) des elastischen Elementes (E) 2,0-18,0 cm beträgt.

2. Hygieneartikel nach Anspruch 1, **dadurch gekennzeichnet, dass** der hintere Schrittbereich (10b) einen an die Quermittelachse (Q) unmittelbar angrenzenden inneren Bereich (10b1) und einen an den inneren Bereich (10b1) anschließenden, sich in Längsrichtung bis zum Rückenteil erstreckenden äußeren Bereich (10b2) aufweist, und wobei sowohl die zweiten faden- oder bandförmigen Elastifizierungsmittel (2), welche entlang dem zweiten Abschnitt (32) der jeweiligen Beinöffnung (8 a, b) angeordnet sind, also auch das elastische Element (E) ausschließlich in dem äußeren Bereich (10b2) angeordnet sind.

3. Hygieneartikel nach Anspruch 2, **dadurch gekennzeichnet, dass** der Abstand (70) in Querrichtung (Q) zwischen dem Rand (5) des Absorptionskerns (22) und einer jeweiligen Beinöffnung in dem äußeren Bereich (10b2) zumindest 5 cm, insbesondere zumindest 7 cm und weiter insbesondere zumindest 9 cm beträgt.

4. Hygieneartikel nach Anspruch 1, 2 oder 3, **dadurch gekennzeichnet, dass** die Länge der Längskomponente (LL) des elastischen Elementes (E) 4,5-19,0 cm, insbesondere 6,5-17,0 cm und weiter insbesondere 7,5-15,0 cm beträgt.

5. Hygieneartikel nach Anspruch 1, 2, 3 oder 4, **dadurch gekennzeichnet, dass** die Länge der Querkomponente (QQ) des elastischen Elementes (E) 4,0-15,0 cm, insbesondere 5,0-13,0 cm und weiter insbesondere 6,0-12,0 cm beträgt.

6. Hygieneartikel nach einem der Ansprüche 2-5, **dadurch gekennzeichnet, dass** der innere Bereich (10b1) des hinteren Schrittbereiches (10b) einer jeweiligen Beinöffnung (8 a, b) dritte faden- oder bandförmige Elastifizierungsmittel (3) aufweist, welche entlang einem dritten Abschnitt (31) der jeweiligen Beinöffnung (8 a, b) angeordnet sind.

7. Hygieneartikel nach einem der vorstehenden Ansprüche, **dadurch gekennzeichnet, dass** die ersten und/oder zweiten und/oder dritten faden- oder bandförmigen Elastifizierungsmittel (1, 2, 3) ausgehend von einer ersten Beinöffnung (8a) den Schrittbereich quer unterhalb des Absorptionskerns (22) traversieren, um auf der gegenüberliegenden Seite einen zur Mittellängsachse (L) spiegelbildlichen Verlauf entlang der weiteren zweiten Beinöffnung (8b) zu nehmen.

8. Hygieneartikel nach Anspruch 7, **dadurch gekennzeichnet, dass** die unterhalb des Absorptionskerns (22) verlaufenden Elastifizierungsmittel (1, 2, 3) insbesondere durch Anwendung von Hitze und/oder Druck und/oder Lasereinwirkung und/oder Ultraschall und/oder durch Schneiden zumindest abschnittsweise ihrer elastifizierenden Wirkung benommen worden sind.

9. Hygieneartikel nach Anspruch 7 oder 8, **dadurch gekennzeichnet, dass** die unterhalb des Absorptionskerns (22) verlaufenden Elastifizierungsmittel (1, 2, 3) spannungsfrei verlaufen.

10. Hygieneartikel nach einem der vorstehenden Ansprüche, **dadurch gekennzeichnet, dass** die ersten Elastifizierungsmittel (1) von den dritten Elastifizierungsmitteln (3) in Längsrichtung weiter beabstandet sind als die zweiten Elastifizierungsmittel (2) von den dritten Elastifizierungsmitteln (3).

11. Hygieneartikel nach einem der vorstehenden Ansprüche, **dadurch gekennzeichnet, dass** die chassisbildende Hülle (21) im Rückenteil (4) und im Vorderteil (6) und insbesondere auch in einem an das Rückenteil und/oder Vorderteil angrenzenden Bereich des Schrittbereiches (10) in Querrichtung verlaufende band- oder fadenförmige Elastifizierungsmittel (61) aufweist.

12. Hygieneartikel nach Anspruch 11, **dadurch gekennzeichnet, dass** die in Querrichtung verlaufenden band- oder fadenförmigen Elastifizierungsmittel (61) insoweit sie unterhalb des Absorptionskerns (22) verlaufen ihrer elastischen Wirkung benommen sind.

13. Hygieneartikel nach einem der vorstehenden Ansprüche, **dadurch gekennzeichnet dass** beidseits des Absorptionskerns (22) und in dessen Längsrichtung erstreckt Bündchenelemente (7) mit einem elastischen Bündchenabschnitt (9) vorgesehen sind.

14. Hygieneartikel nach einem der vorstehenden Ansprüche, **dadurch gekennzeichnet, dass** der Absorptionskern (22) körpernah von einem Topsheet (13) überfangen und körperfern von einem Backsheet (14) unterfangen ist.

15. Hygieneartikel nach einem der vorstehenden Ansprüche, **dadurch gekennzeichnet, dass** der hintere Schrittbereich (10b) länger ist als der vordere Schrittbereich (10a).

16. Hygieneartikel nach einem der vorstehenden Ansprüche, **dadurch gekennzeichnet, dass** die Summe der Längen (1-L, 2-L, 3-L) der ersten bis dritten Abschnitte (30, 31, 32) einer jeweiligen Beinöffnung (8 a, b) 20-60 cm, insbesondere 25-55 cm, weiter insbesondere 27-50 cm und weiter insbesondere 29-48 cm beträgt.

17. Hygieneartikel nach einem der vorstehenden Ansprüche, **dadurch gekennzeichnet, dass** das Verhältnis der Summe der Längen (1-L, 2-L, 3-L) der ersten bis dritten Abschnitte (30, 31, 32) einer jeweiligen Beinöffnung (8 a, b) zur Umfangslänge einer jeweiligen Beinöffnung höchstens 0,7, insbesondere höchstens 0,6, weiter insbesondere höchstens 0,55 und weiter insbesondere höchstens 0,5 beträgt.

## Claims

1. Disposable absorbent sanitary article (200) for adults in the form of briefs, having an absorbent core (22) and having a chassis-forming outer envelope (21) with a waist edge (20) that forms a waist opening and is continuously closed in the circumferential direction, and having a front part (6) and a rear part (4) that have longitudinal-side edge portions (16, 18), and having a crotch region (10) that is arranged between the front part (6) and rear part (4) and form the leg openings (8a, b), wherein the waist edge (20) that is continuously closed in the circumferential direction and the leg openings (8a, b) are formed by longitudinal-side edge portions (16, 18) of a front part (6) and of a rear part (4) of the chassis-forming outer envelope (21) being joined together by the producer, and having a longitudinal centre axis (L) extending in a longitudinal direction and having a transverse centre axis (Q) extending in a transverse direction through the middle of the crotch region (10), said transverse centre axis (Q) dividing the crotch region into a front crotch region (10a) and a rear crotch region (10b), and wherein each leg opening (8a, b) has first thread-form or band-form elasticating means (1) in the front crotch region (10a), said elasticating means (1) being arranged along a first portion (30) of each leg opening, and wherein the rear crotch region (10b) has second thread-form or band-form elasticating means (2), which are arranged along a second portion (32) of each leg opening (8a, b), and wherein the rear crotch region (10b) has an elastic element (E) between each longitudinal edge (5) of the absorbent core (22) and each leg opening (8a, b), said elastic element (E) being arranged in a direction, starting from a region close to the leg opening, that has both a transverse component (QQ) in the direction of the longitudinal centre axis and a longitudinal component (LL) in the direction of the rear part (4), **characterized in that** the elastic element (E) is formed by a further extension of the second thread-form or band-form elasticating means (2) by the latter initially extending along the second portion (32) of each leg opening in the direction of the transverse centre axis (Q) and then extending from the region close to the leg opening, forming a U- or V-shaped configuration, in a direction that has both a transverse component QQ in the direction of the longitudinal centre axis (L) and a longitudinal component LL in the direction of the rear part (4), wherein the length of the longitudinal component (LL) of the elastic element (E) is 2.5-21.0 cm and the length of the transverse component (QQ) of the elastic element (E) is 2.0-18.0 cm.

2. Sanitary article according to Claim 1, **characterized in that** the rear crotch region (10b) has an inner region (10b1) immediately adjoining the transverse centre axis (Q) and an outer region (10b2) adjoining the inner region (10b1) and extending in the longitudinal direction as far as the rear part, and wherein both the second thread-form or band-form elasticating means (2), which are arranged along the second portion (32) of each leg opening (8a, b), and the elastic element (E) are arranged only in the outer region (10b2).

3. Sanitary article according to Claim 2, **characterized in that** the distance (70) in the transverse direction (Q) between the edge (5) of the absorbent core (22) and each leg opening in the outer region (10b2) is at least 5 cm, in particular at least 7 cm and more particularly at least 9 cm.

4. Sanitary article according to Claim 1, 2 or 3, **characterized in that** the length of the longitudinal component (LL) of the elastic element (E) is 4.5-19.0 cm, in particular 6.5-17.0 cm and more particularly 7.5-15.0 cm.

5. Sanitary article according to Claim 1, 2, 3 or 4, **characterized in that** the length of the transverse component (QQ) of the elastic element (E) is 4.0-15.0 cm, in particular 5.0-13.0 cm and more particularly 6.0-12.0 cm.

6. Sanitary article according to one of Claims 2-5, **characterized in that** the inner region (10b1) of the rear crotch region (10b) of each leg opening (8a, b) has third thread-form or band-form elasticating means (3), which are arranged along a third portion (31) of each leg opening (8a, b).

7. Sanitary article according to one of the preceding claims, **characterized in that** the first and/or second and/or third thread-form or band-form elasticating means (1, 2, 3), starting from a first leg opening (8a), cross the crotch region transversely beneath the absorbent core (22) in order to adopt a mirror-symmetric course with respect to the longitudinal centre axis (L) along the further, second leg opening (8b) on the opposite side.

8. Sanitary article according to Claim 7, **characterized in that** the elasticating means (1, 2, 3) extending beneath the absorbent core (22) have had their elasticating effect at least partially removed in particular by application of heat and/or pressure and/or laser exposure and/or ultrasound and/or by cutting.

9. Sanitary article according to Claim 7 or 8, **characterized in that** the elasticating means (1, 2, 3) extending beneath the absorbent core (22) extend in a tension-free manner.

10. Sanitary article according to one of the preceding claims, **characterized in that** the first elasticating means (1) are further away from the third elasticating means (3) in the longitudinal direction than the second elasticating means (2) are from the third elasticating means (3).

11. Sanitary article according to one of the preceding claims, **characterized in that** the chassis-forming envelope (21) has band-form or thread-form elasticating means (61) extending in a transverse direction in the rear part (4) and in the front part (6) and in particular also in a region of the crotch region (10) that adjoins the rear part and/or front part.

12. Sanitary article according to Claim 11, **characterized in that** the band-form or thread-form elasticating means (61) extending in the transverse direction, inasmuch as they extend beneath the absorbent core (22), have had their elastic effect removed.

13. Sanitary article according to one of the preceding claims, **characterized in that** cuff elements (7) having an elastic cuff portion (9) are provided on both sides of the absorbent core (22) in a manner extending in the longitudinal direction thereof.

14. Sanitary article according to one of the preceding claims, **characterized in that** the absorbent core (22) is overlaid with a top sheet (13) next to the body and underlaid with a back sheet (14) away from the body.

15. Sanitary article according to one of the preceding claims, **characterized in that** the rear crotch region (10b) is longer than the front crotch region (10a).

16. Sanitary article according to one of the preceding claims, **characterized in that** the sum of the lengths (1-L, 2-L, 3-L) of the first to third portions (30, 31, 32) of each leg opening (8a, b) is 20-60 cm, in particular 25-55 cm, more particularly 27-50 cm and more particularly 29-48 cm.

17. Sanitary article according to one of the preceding claims, **characterized in that** the ratio of the sum of the lengths (1-L, 2-L, 3-L) of the first to third portions (30, 31, 32) of each leg opening (8a, b) to the circumferential length of each leg opening is at most 0.7, in particular at most 0.6, more particularly at most 0.55 and more particularly at most 0.5.

## Revendications

1. Article hygiénique absorbant jetable (200) en forme de culotte pour adultes, comprenant un cœur absorbant (22) et une enveloppe extérieure (21) formant un châssis avec un bord au niveau des hanches (20) formant une ouverture pour les hanches et fermé de manière continue dans la direction périphérique, et avec une partie avant (6) et une partie arrière (4) présentant des portions de bords latéraux longitudinaux (16, 18), et avec une région d'entrejambe (10) entre la partie avant (6) et la partie arrière (4), formant les ouvertures pour les jambes (8a,b), le bord au niveau des hanches (20) fermé de manière continue dans la direction périphérique et les ouvertures pour les jambes (8a,b) étant formés en reliant ensemble, chez le fabricant, les portions de bords latéraux longitudinaux (16, 18) d'une partie avant (6) et d'une partie arrière (4) de l'enveloppe extérieure (21) formant le châssis, et avec un axe longitudinal médian (L) s'étendant dans la direction longitudinale et avec un axe médian transversal (Q) s'étendant dans la direction transversale à travers le centre de la région d'entrejambe (10), qui divise la région d'entrejambe en une région d'entrejambe avant (10a) et une région d'entrejambe arrière (10b), et une ouverture respective pour les jambes (8a,b) dans la région d'entrejambe avant (10a) présentant des premiers moyens d'élastification en forme de fils ou de bandes (1) qui sont disposés le long d'une première portion (30) de l'ouverture respective pour les jambes, et la région d'entrejambe arrière (10b) présentant des deuxièmes moyens d'élastification en forme de fils ou de bandes (2) qui sont disposés le long d'une deuxième portion (32) de l'ouverture respective pour les jambes (8a,b), et la région d'entrejambe arrière (10b) présentant entre le bord longitudinal respectif (5) du cœur absorbant (22) et l'ouverture respective pour les jambes (8a,b) un élément élastique (E) qui est disposé à partir d'une région proche de l'ouverture pour les jambes dans une direction qui présente à la fois une composante transversale (QQ) dans la direction de l'axe longitudinal médian et une composante longitudinale (LL) dans la direction de la partie arrière (4), **caractérisé en ce que** l'élément élastique (E) est formé par une étendue supplémentaire des deuxièmes moyens d'élastification en forme de fils ou de bandes (2) par le fait que ceux-ci s'étendent d'abord le long de la deuxième portion (32) de l'ouverture respective pour les jambes dans la direction de l'axe médian transversal (Q) et s'étendent ensuite en formant une configuration en forme de U ou en forme de V à partir de la région proche de l'ouverture pour les jambes dans une direction qui présente à la fois une composante transversale QQ dans la direction de l'axe longitudinal médian (L) et une composante longitudinale LL dans la direction de la partie arrière (4), la longueur de la composante longitudinale (LL) de l'élément élastique (E) mesurant 2,5 à 21,0 cm et la longueur de la composante transversale (QQ) de l'élément élastique (E) mesurant 2,0 à 18,0 cm.

2. Article hygiénique selon la revendication 1, **caractérisé en ce que** la région d'entrejambe arrière (10b) présente une région intérieure (10b1) directement adjacente à l'axe médian transversal (Q) et une région extérieure (10b2) se raccordant à la région intérieure (10b1), s'étendant dans la direction longitudinale jusqu'à la partie arrière, et à la fois les deuxièmes moyens d'élastification en forme de fils ou de bandes (2), qui sont disposés le long de la deuxième portion (32) de l'ouverture respective pour les jambes (8a,b), et l'élément élastique (E), étant disposés exclusivement dans la région extérieure (10b2).

3. Article hygiénique selon la revendication 2, **caractérisé en ce que** la distance (70) dans la direction transversale (Q) entre le bord (5) du cœur absorbant (22) et une ouverture respective pour les jambes dans la région extérieure (10b2) mesure au moins 5 cm, en particulier au moins 7 cm et plus particulièrement au moins 9 cm.

4. Article hygiénique selon la revendication 1, 2 ou 3, **caractérisé en ce que** la longueur de la composante longitudinale (LL) de l'élément élastique (E) mesure 4,5 à 19,0 cm, en particulier 6,5 à 17,0 cm et plus particulièrement 7,5 à 15,0 cm.

5. Article hygiénique selon la revendication 1, 2, 3 ou 4, **caractérisé en ce que** la longueur de la composante transversale (QQ) de l'élément élastique (E) mesure 4,0 à 15,0 cm, en particulier 5,0 à 13,0 cm et plus particulièrement 6,0 à 12,0 cm.

6. Article hygiénique selon l'une quelconque des revendications 2 à 5, **caractérisé en ce que** la région intérieure (10b1) de la région d'entrejambe arrière (10b) d'une ouverture respective pour les jambes (8a,b) présente des troisièmes moyens d'élastification en forme de fils ou de bandes (3) qui sont disposés le long d'une troisième portion (31) de l'ouverture respective pour les jambes (8a,b).

7. Article hygiénique selon l'une quelconque des revendications précédentes, **caractérisé en ce que** les premiers et/ou deuxièmes et/ou troisièmes moyens d'élastification en forme de fils ou de bandes (1, 2, 3) s'étendent à partir d'une première ouverture pour les jambes (8a) à travers la région d'entrejambe transversalement en dessous du cœur absorbant (22) afin de se poursuivre du côté opposé avec une allure à symétrie spéculaire par rapport à l'axe longitudinal médian (L) le long de la deuxième ouverture supplémentaire pour les jambes (8b).

8. Article hygiénique selon la revendication 7, **caractérisé en ce que** les moyens d'élastification (1, 2, 3) s'étendant en dessous du cœur absorbant (22) sont dépourvus au moins en partie de leur effet élastique en particulier par application de chaleur et/ou de pression et/ou par l'action d'un laser et/ou d'ultrasons et/ou par découpage.

9. Article hygiénique selon la revendication 7 ou 8, **caractérisé en ce que** les moyens d'élastification (1, 2, 3) s'étendant en dessous du noyau absorbant (22) s'étendent sans tension.

10. Article hygiénique selon l'une quelconque des revendications précédentes, **caractérisé en ce que** les premiers moyens d'élastification (1) sont plus espacés des troisièmes moyens d'élastification (3) dans la direction longitudinale que les deuxièmes moyens d'élastification (2) sont espacés des troisièmes moyens d'élastification (3).

11. Article hygiénique selon l'une quelconque des revendications précédentes, **caractérisé en ce que** l'enveloppe formant un châssis (21) dans la partie arrière (4) et dans la partie avant (6) et notamment également dans une région de la région d'entrejambe (10) adjacente à la partie arrière et/ou à la partie avant, présente des moyens d'élastification (61) en forme de bandes ou de fils s'étendant dans la direction transversale.

12. Article hygiénique selon la revendication 11, **caractérisé en ce que** les moyens d'élastification (61) en forme de bandes ou de fils s'étendant dans la direction transversale, dans la mesure où ils s'étendent en dessous du noyau absorbant (22), sont dépourvus de leur effet élastique.

13. Article hygiénique selon l'une quelconque des revendications précédentes, **caractérisé en ce que** des éléments d'élastiques (7) avec une portion d'élastique (9) élastique, sont prévus de part et d'autre du noyau absorbant (22) et s'étendent dans sa direction longitudinale.

14. Article hygiénique selon l'une quelconque des revendications précédentes, **caractérisé en ce que** le noyau absorbant (22) est recouvert à proximité du corps par une feuille supérieure (13) et est recouvert par le dessous à distance du corps par une feuille de support (14).

15. Article hygiénique selon l'une quelconque des revendications précédentes, **caractérisé en ce que** la région d'entrejambe arrière (10b) est plus longue que la région d'entrejambe avant (10a).

16. Article hygiénique selon l'une quelconque des revendications précédentes, **caractérisé en ce que** la somme des longueurs (1-L, 2-L, 3-L) des première à troisième portions (30, 31, 32) d'une ouverture respective pour les jambes (8a,b) mesure 20 à 60 cm, en particulier 25 à 55 cm, plus particulièrement 27 à 50 cm et plus particulièrement 29 à 48 cm.

17. Article hygiénique selon l'une quelconque des revendications précédentes, **caractérisé en ce que** le rapport de la somme des longueurs (1-L, 2-L, 3-L) des première à troisième portions (30, 31, 32) d'une ouverture respective pour les jambes (8a,b) par rapport à la longueur périphérique d'une ouverture respective pour les jambes vaut au maximum 0,7, en particulier au maximum 0,6, plus particulièrement au maximum 0,55 et encore plus particulièrement 0,5.
